**Europäisches Patentamt**

**European Patent Office**

**Office européen des brevets**

(11) Publication number: **0 083 973**

**A1**

# (12) EUROPEAN PATENT APPLICATION

(21) Application number: **83300061.5**

(22) Date of filing: **06.01.83**

(51) Int. Cl.³: **A 61 B 10/00**

(30) Priority: **07.01.82 US 337899**
**07.01.82 US 337908**
**07.01.82 US 337872**

(43) Date of publication of application:
**20.07.83 Bulletin 83/29**

(84) Designated Contracting States:
**DE FR GB IT NL SE**

(71) Applicant: **TECHNICARE CORPORATION**
**90 Inverness Circle East**
**Englewood Colorado 80112(US)**

(72) Inventor: **Trimmer, William S. N.**
**58 Riverview Terrace**
**Belle Mead, N. J. 08502(US)**

(72) Inventor: **Gardineer, Bayard, Jr.**
**6 Pine Brae Drive**
**Skillman, N. J. 08558(US)**

(72) Inventor: **Hadjicostis, Andreas**
**999 Hidden Lake Drive 22A**
**North Brunswick, N. J. 08902(US)**

(72) Inventor: **Vilkomerson, David H. R.**
**2 Carter Brook Lane, RD4**
**Princeton, N. J. 08540(US)**

(72) Inventor: **Robbins, Wayne C.**
**8158 East Phillips Circle**
**Englewood Colorado 80112(US)**

(74) Representative: **Colgan, Stephen James et al,**
**CARPMAELS & RANSFORD 43 Bloomsbury Square**
**London WC1A 2RA.(GB)**

(54) Ultrasound probe locator.

(57) Techniques for locating the tip of a hollow needle probe are disclosed. The needle (101) contains a conduit for conducting ultrasonic energy to energy receiving means outside the body. In one embodiment the conduit comprises a sonically conductive rod (102). When directional information is desired, a plurality of conduits may be used, or a single conduit including a plurality of detector transducers located near the needle tip. A system for irradiating the probe is shown and a method for use of the present invention is provided. The conduit may also be insulated from the needle by a packing of microspheres.

Fig.1.

## Ultrasound Probe Locator

The present invention relates to the field of pulse-echo ultrasound imaging systems which are used with probes which are inserted into or through body tissue and maneuvered so that a selected portion of that probe is within the imaged area. Aspiration biopsy is one example of such a procedure.

In aspiration biopsy, a small, hollow needle is inserted directly into the body to a desired point, whereupon a tissue sample is withdrawn, such as by vacuum aspiration. The needle is then withdrawn, but because of its relatively insignificant diameter, wound closure occurs normally by muscular and tissue tension, generally without the need for sutures, cauterization, or the like. For optimal effectiveness, aspiration biopsy techniques require that the tip of the needle be precisely accurately placed at the location of the tissues to be sampled. When properly performed, these techniques are safe, effective, and minimally traumatic.

Other common diagnostic and therapeutic procedures involving the maneuvering of a probe through body tissues include various catheterization procedures. In these procedures, a catheter is maneuvered through a vein, artery, or other duct to a desired internal location. Such procedures include arterial angioplasty, thrombus removal, bile duct clearage, etc.

Various radiological (i.e. X-ray fluoroscopic) techniques have been utilized for ascertaining or estimating the location of tissue to be treated, sampled or diagnosed, and for aiding in the maneuvering of a given probe to its desired body location. These radiological techniques not only suffer from dimensional/accuracy limitations, but also from other inherent drawbacks related to the radiation doese involved in performing such procedures.

More recently, a variety of ultrasound imaging techniques have been suggested, particularly for in vivo imaging of relatively deep soft body tissue. Such ultrasound techniques include conventional A-mode, B-mode, and C-mode approaches. Because of their superior imaging capability with minimal hazard or risk to the patient, such techniques have achieved prominent use, particularly for needle location during aspiration biopsy techniques.

In one class of prior art systems, exemplified by U.S. Patents 3,556,079 (Omizo) and 4,429,539 (Vilkomerson et al), a transducer element at the skin and another one within or at the tip of the needle correspond with one another to precisely locate the needle in the image field. In such systems, the needle is normally insertable into the body at an angle and in a direction which is completely independent from the angle and the position of the pulse-echo ultrasound imaging system. Accordingly, while such systems are effective at imaging the needle once the tip is disposed within the ultrasonically illuminated scan plane (imaging field), difficulties may be encountered in locating the needle in the scan plane for illumination and imaging.

These prior art systems have experienced a certain degree of commercial success, and are normally effective for their intended uses. Perhaps the lease efficient of these systems are those which require parallel orientation of the propagating ultrasound energy and the needle, because of their failure to yield either clear composite images or accurate needle depth representation. While the systems of U.S. Patent 4,429,539 (Vilkomerson et al) are among the most effective, such systems do generally require special electronics and compact, expensive transducer elements which are mountable at the needle tip and removable through the biopsy needle.

The present invention represents an improvement in the Vilkomerson et al type imaging systems. This improvement aids in positioning a probe within the scan plane by providing directional information to the user concerning the relative scan-plane and probe locations. The present invention thus provides a novel system for ensuring that the tip of a probe, such as a biopsy needle or catheter, may be readily positioned within the scan plane of an external pulse-echo imaging system during the insertion of the probe into or through body tissue which is to be subjected to a therapeutic or diagnostic procedure.

The principles of the present invention are premised upon utilization of a stylet-like element for sensing sonic pulses in the body and conveying those pulses out through the needle, where they are converted to electrical energy. Thus, in a first embodiment of the present invention, a coaxial needle probe carries a coaxial conduit rod during insertion, which terminates in the region of the needle tip, as desired either just outside, at, or within the needle tip. The stylet conduit is coupled to a transducer outside the body, such that ultrasound pressure waves which are conducted through the needle and out of the body are converted to electrical energy. Hence, the only portion of the apparatus which penetrates and/or contacts body tissues are the electrically passive, essentially disposable and easily sterilizable needle and stylet portions.

In a second embodiment, the stylet--sonic conduit is fashioned of stainless steel, and terminates in a sonic matching layer. The stylet--conduit is isolated from the interior surface of the needle either by spacing or by insulating materials.

In accordance with the principles of a further aspect of the present invention, a packing of hollow, plastic microspheres substantially completely sonically isolates the stylet from the interior surface of the needle sheath. Hence, pressure waves originating at the stylet tip are conveyed back through the needle and to an external transducer/utilization apparatus, free of the danger of the significant transfer of those pressure waves to the needle itself. In a third embodiment, the stylet is of stainless steel and conventional, rod-shaped construction (either finished smooth at its face or carrying a matching layer), is first coated, either by dipping or spin coating, with a low viscosity nonconductive epoxy film, and then immersed into a vessel filled with the plastic microspheres, which adhere to the epoxy. After curing of the epoxy, the stylet is inserted into the needle, thereby shearing off the excessive microspheres, and insuring a well-packed, integral acoustic insulation between the stylet and the interior of the needle.

In accordance with the principles of another embodiment of the present invention, a plurality of detector-transducers are spacially oriented at distinct probe locations, and are discretely connected to circuit means through the coaxial needle for sensing the sequence of arrivals of ultrasound waves at such locations. Such sequences are indicative of the probe position with respect to the scan plane, and are used to indicate the direction in which the scan plane or probe should be moved in order to cause the probe to fall within the scan plane.

In the drawings:

FIGURE 1 shows an illustrative embodiment of the principles of the present invention, including needle, stylet, and transducer means;

FIGURE 2 shows a partial cutaway, magnified and distorted for convenience of explanation, with respect to the embodiment of FIGURE 1;

FIGURE 3 shows an isometric illustration of the point region of the embodiment of FIGURE 1;

FIGURES 4 and 5 show alternative embodiments of stylet conduits in accordance with the principles of the present invention;

FIGURES 6, 7A, 7B, 8A and 8B show alternative embodiments of the principles of the present invention;

FIGURES 9 and 10 illustrate a technique for insulating the needle from the stylet;

FIGURES 11 and 12 illustrate an arrangement for determining the directional position of a needle; and

FIGURE 13 illustrates further details of the directional location system of FIGURES 11 and 12.

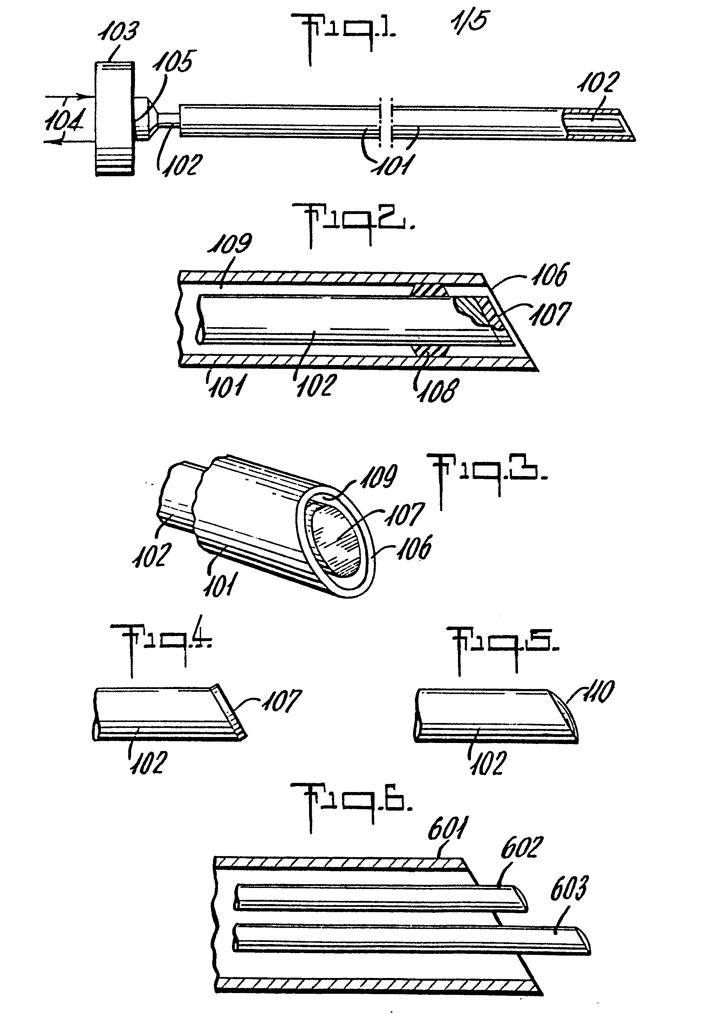

Referring then, to the instant figures, there are shown various illustrative and preferred embodiments of the principles of the present invention. In FIGURE 1, a hollow needle 101, shown foreshortened for convenience, carries therein a stylet 102 which extends from the proximate end of the needle (i.e. the end which stays outside the body) through the needle to the distal end thereof (i.e. the end which penetrates the body). In FIGURE 1, the distal end of the needle 101 is cut away, revealing the stylet 102 which extends approximately to the distal opening of the needle 101. At the proximate end of the needle 101, the stylet 102 flares outwardly and, at a surface 105 abuts a transducer 103. As is conventional in the art, impinging sonic pulses at surface 105 excite a piezoelectric response in the transducer 103, and electrical signals are generated, as indicated symbolically by connections 104. It has been found that physically abutting complementary surfaces at 105 are adequate in accordance with the principles of the present invention, but that the conduct of sonic energy from the stylet conduit 102 to the transducer 103 may be facilitated by utilization of gel, oil, or the like coupling media standard in the art, intermediate the stylet 102 and the transducer 103. In other forms of the present invention, the stylet 102, rather than flaring outwardly as shown in FIGURE 1, tapers and is forced directly into the surface of the transducer 103. Numerous other configurations are deemed well within the purview of the principles of the present invention, provided simply that they allow the sonic energy from the stylet 102 to be coupled to the transducer 103 for adequate piezoelectric response thereat. Additionally, the transducer 103 may be the self same transducer which is utilized for the basic imaging system.

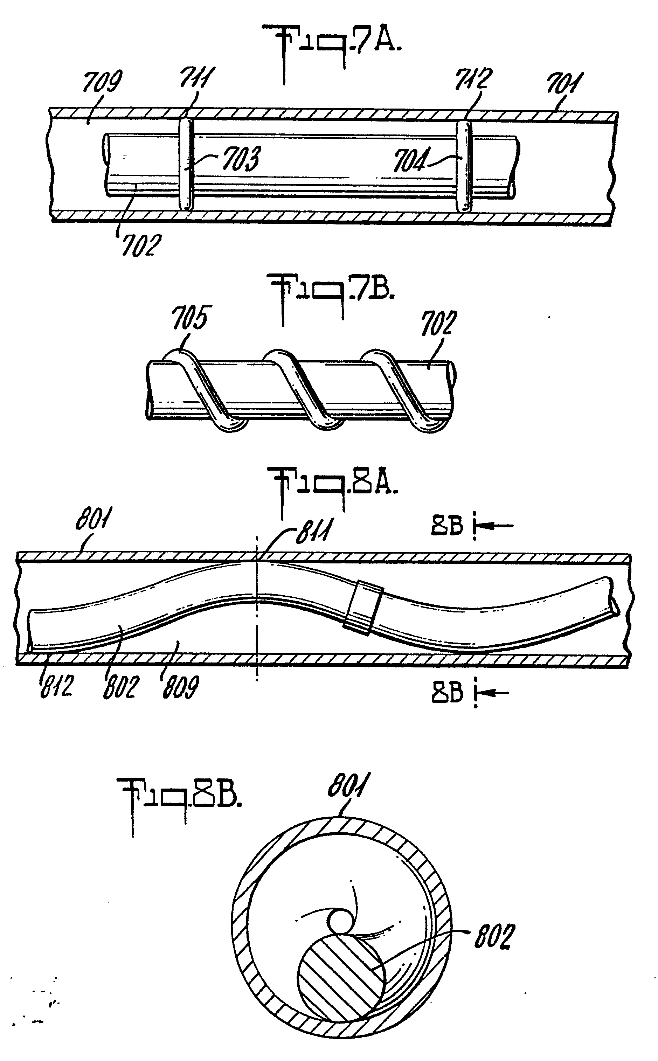

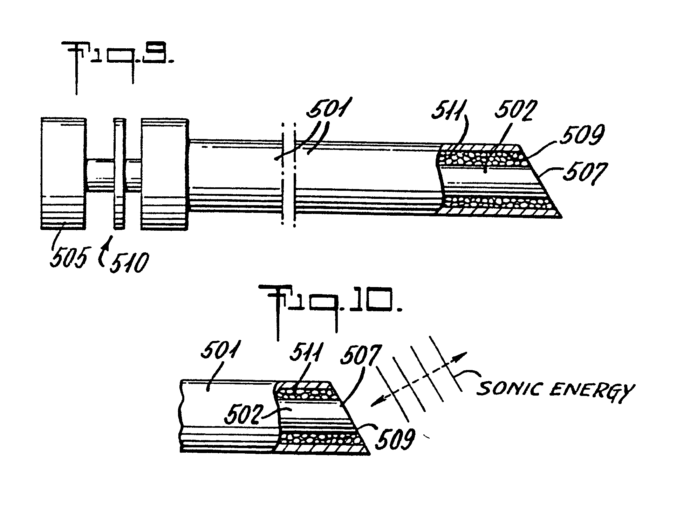

Referring next to FIGURE 2, there is shown an enlarged view of the cutaway distal portion of the apparatus of FIGURE 1. In FIGURE 2, the needle 101 is cut away, but the stylet 102 is not. The stylet conduit 102 is generally coaxial with the needle 101, with an annular space 109 being formed to isolate the stylet 102 physically from the needle 101. It will be noted that alternative embodiments of the present invention will feature various spacers between the inner surface of the
needle 101 and the stylet 102, in order to maintain
spatial isolation therebetween. It is to be noted,
however, that the principles of the present invention are
generally operative even in the absence of any spacer
between the stylet 102 and the inner surface of the needle
101, and that as the needle is inserted into the body,
some small amount of bending will occur, and some minimal
amount of contact will occur between the stylet 102 and
the surface of the needle 101, but that such contact will
not obviate the effectiveness of the principles of the
present invention. In fact, some such contact may even be
beneficial, tending to damp radial or transverse vibration
in the stylet, and hence promote longitudinal propagation
of the sensed ultrasound pressure waves. In FIGURE 2, the
stylet 102 is shown terminating in a matching layer 107,
which is an optional but highly desirable feature in order
to enhance the efficiency of translating the sonic pulses
in the body into sonic pressure waves passing through the
stylet conduit 102. The embodiment of FIGURE 2 also shows
an annulus of flexible material, for example of commercially available silicone caulking materials, which not
only provides spacing betwen the stylet 102 and the needle
101, but more importantly serves as a gasket to prevent
backflow of fluids into the needle. For ease of understanding, FIGURE 3 shows a schematic isometric view of the
distal point of the needle 101 and the stylet--conduit
102, in a form which has not been cut away.

It will be noted that the needle 101 itself, supplemented
by the annular space 109, forms a protective sheath for
the stylet 102, partially reflecting incident energy, and
absorbing and substantially dissipating the balance
thereof. Hence, the elongated stylet 102 within the
needle 101 acquires sonic energy essentially only at the
tip 107 thereof.

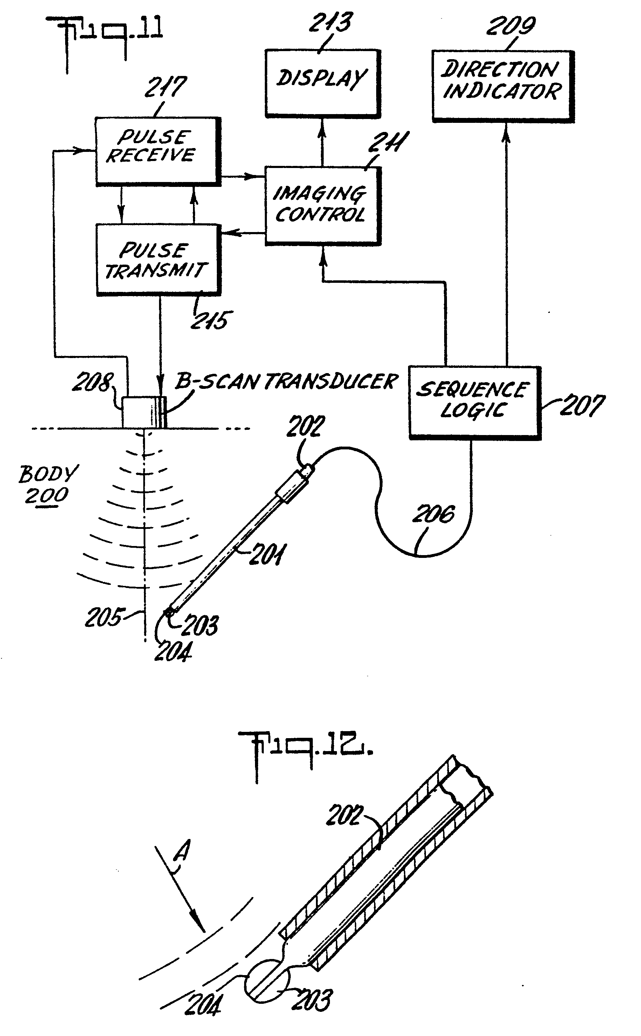

It will be noted from the embodiments of FIGURES 1 through
3 that the needle 106 and the stylet 102 are beveled at
their terminus, at an angle of approximately 45°. Such is
a rather conventional configuration, which is convenient
for application in accordance with the principles of the
present invention. The angle of beveling is not critical,
however, inasmuch as the stylet--conduit 102 need not
repose within the point of the needle 101, as is shown in
the drawing. Indeed, the stylet 102 may be further
extended beyond the needle point, and even rotated about
in order to facilitate the sensing of sonic pulses. In a
preferred embodiment, the needle 101 and the stylet--
conduit 102 are both fashioned of stainless steel, while
the matching layer 107 is fashioned of one of the numerous
plastic or glass materials in conventional use for
matching in the construction of active ultrasound
transducers. For example, one such plastic is the one
sold commercially under the trade designation "MF114" from
Emerson-Cuming Co. In embodiments which rely on an air
gap 109, such a gap of .001 inches will generally be
adequate; in embodiments which utilize some substance in
the area 109, distances equivalent to a fraction of a
sonic wavelength, for example .01 inches, may be utilized.
In preferred embodiments, the matching layer 107 will be
a quarter wavelength in thickness and may in fact be constituted of a composite or sandwich layering of several
matching materials. As is shown in FIGURES 2 and 4, the
matching layer 107 may be disc-schaped; alternatively it
may be a rounded dome shape as shown by element 110 in
FIGURE 5.

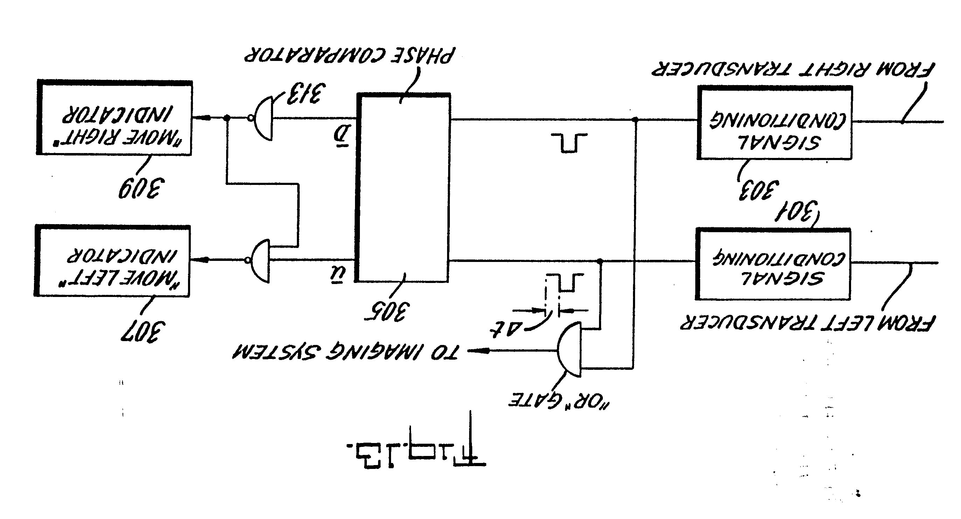

Referring to FIGURE 6, there is shown an alternative
embodiment wherein a larger bore needle 601, which can
accommodate plural stylet--conduits 602, 603, and the
like. In such a system, the plural stylet--conduits 602
and 603 will, in the body, have their distal ends spatially offset from one another, and hence will be impinged by each given sonic wavefront at different times. It is thereby possible, based on the time delay therebetween, to determine sonic rate of propagation, direction of propagation, Doppler factors, and the like.

In use, the principles of the present invention may be applied with advantage to either the receive-superposition or the receive-transmit modes of operation as taught by Vilkomerson et al. That is, received signals may be processed outside the body and combined by superposition with the image field, or the transducer 103 may be utilized to generate a pulse which is carried down through the stylet 102 and thence out into body tissues. In the latter event, it may be desirable to produce such a return pulse after receipt of an imaging pulse. Alternatively, it may be more beneficial from the standpoint of overall system utilization to fire the stylet transducer 103 simultaneously with the firing of the imaging system transducer. Since the imaging pulse-echo process will entail a round trip propagation delay (e.g. 300 microseconds), generating a pulse at the same time from the stylet will insure that the needle point locator signal will be "heard" by the imaging system after only half the time (e.g. 150 microseconds), hence at a time when the imaging system electronics are otherwise quiescent and not otherwise gainfully employed. The system processor then will, simply through suitable delay of processing, produce the composite image including the pulse-echo signal with the point locator signal.

Referring next to FIGURES 7A, 7B, 8A and 8B, there are shown alternative embodiments which are intended to facilitate effective wave propagation up and down the stylet. In brief, it may be desirable to provide spacer mechanisms periodically along the length of the stylet, so that the wavefront will not dissipate along the stylet due
to spurious, irregular translation of the stylet. In
FIGURE 7A, periodic ridges 703, 704 etc. act as spacers
to insure reasonable maintenance of the gap 709 between
the needle 701 and the stylet 702. In practice, the
ridges 703, 704 etc. may be integral annular extensions
from the stylet 702, or may be a continuous helical ridge
705 as shown in FIGURE 7B. Likewise, the annuli 703 and
704, or the heliz 705 may be separately affixed over the
stylet 702. FIGURE 8A and FIGURE 8B which is a transverse
section thereof, shows an alternative configuration
wherein the stylet 802 is itself configured as an extended
helix, periodically contacting the needle 801 and thereby
being physically secure therein.

In the embodiments of FIGURES 7A and 7B, and that of
FIGURES 8A and 8B, the periodic, nearly point contacts
711, 712, 811, 812, etc., provide an "anti-jiggle"
security for the stylets 702, 802, respectively, but do
not appreciably affect the longitudinal propagation of
energy along the stylets.

Referring, then, to FIGURES 9 and 10, there is shown a
further embodiment of the principles of the present
invention. In paricular, FIGURE 9 shows a partial lateral
cutaway of the embodiment, whereas FIGURE 10 shows only
the distal end of the apparatus, which is inserted into
body. In particlar, a tubular sheath 501, for example a
stainless steel aspiration needle having an inside diameter in the range of one-half millimeters or less, has a
proximal end which is grasped in conventional fashion for
insertion into the body, and a distal end 509 beveled at
some angle, for example 45°. Within the needle 501 is a
rod-like, generally coaxial stylet (i.e. sonic conduit)
502 which terminates at a surface 507 in the region of the
distal end 509 of the needle 501. The distal surface 507 of the stylet 502 may be simply of polished metal, or may include a matching layer such as that shown in FIGURE 2.

The stylet 502 optionally carries a collar or "stop" 510 which limits its travel within the needle 501, and hence also establishes preferred positions for the distal end 507 of the stylette 502. At its extreme proximal end, the stylet 502 defines a broader portion 505 for coupling to a transducer apparatus.

In accordance with the principles of the present invention, the space intermediate the stylet 502 and the inner surface of the needle 501 is filled with hollow, plastic microspheres, which provide acoustic insulation of the stylet from its surrounding needle 501. In preferred embodiments, these hollow plastic microspheres, having an approximate diameter of 0.8 millimeters, substantially completely pack the space between the stylet 502 and the needle 501.

A preferred fashion for assembling the embodiment of FIGURES 9 and 10 is as follows. The stylet, which preferably is formed of stainless steel material, is provided with a coating of a film of low viscosity, nonconductive epoxy, for example in the range of .005 millimeters in thickness. This may be done in conventional fashion either by dip coating or spin coating the epoxy onto the stylet. Thereupon, the coated stylet is dipped into a vessel which contains the loose, hollow plastic microspheres. These then adhere to the epoxy, in fact in quite permanent fashion upon curing thereof. This process may be repeated a number of times, as desired, with the epoxy being allowed fully to cure with the plastic microspheres adherent thereon. Then, the coated stylet is carefully inserted wither into the needle directly, or into a tube which conforms to the size of the needle into which the stylet will be applied. Excess microspheres thereby are sheared from the aggregate on the stylet, and there results a well-formed stylet 502 coated with microspheres 511. It will be noted that the excellent acoustic insulation achieved in accordance with the principles of the present invention allows for improved transmission, as well as reception capacity. That is, in accordance with the principles of the present invention, sonic energy coupled to the stylet at 505 will be carried with very low degrees of energy loss down through the stylet 502, and emitted into the surrounding tissue via the distal surface 507 of the stylet 502.

In a preferred embodiment, the epoxy used to bond the microspheres to the stylet is that commercially available from Tra-Con, Inc. under the trade designation Tra-Bond 2113, which, as is known in the art, is a clear, low viscosity epoxy adhesive. In such preferred embodiment, the microspheres are those commercailly available and generally known as microballoons, for example those sold by Emerson & Cuming, Inc. under the trade designation Eccospheres, which have a size tolerance of ± 20%.

An alternative embodiment to that shown in FIGURE 6, in which a sonic wavefront is detected at different times and the interval between the times of detection is measured to establish factors such as rate and direction of propagation and Doppler characteristics, is shown in FIGURES 11 and 12. Referring to FIGURE 11, an aspiration needle system useful in accordance with the principles of the present invention is illustrated. In this embodiment, the probe comprises a conventional hollow aspiration needle 201 which is hollow in the center and useful for vacuum aspiration of tissue samples once the needle is in place. In other embodiments, the aspiration needle may be replaced with a catheter when the probe is to be inserted through an existing body opening such as an artery, duct or canal. In the embodiment of FIGURE 11, a the hollow axial channel formed by needle 201 is useful in accordance with the present invention to receive an elongated support which carries a plurality of small, essentially point source, omni-directional transducers 203 and 204 which are disposed on opposite sides of the tip of an elongated support 202. Elongated support 202 also contains appropriate leads (not shown) from the transducers 203 and 204 to external transponder apparatus. Accordingly, the needle assembly of this embodiment may be inserted directly into human tissue as the needle 201 conventionally would be done, whereby the transducers 203 and 204 will, in accordance with the principles of the present invention, be utilized to indicate the location of the tip of the needle with respect to the scan plane produced by an external pulse-echo ultrasound imaging system.

Small omni-directional transducers which function essentially as point sources/receivers are commercially available, and typically are hemispherical in shape, as shown in FIGURE 12. It will be appreciated that the actual size of the transducer 203 and its support 202 will be determined by the dimensions of the needle or catheter with which such transducers are used. It is anticipated that upon appropriate positioning fo the needle or other probe within the body tissue being scanned, the entire mechanism 202, 203 and 204 may be withdrawn from the needle or other probe to thereby permit the interior of the probe to be used in another manner, as for example to aspirate or inject materials therethrough. Hence, the support 202 for the transducers 203 and 204 functions not only as a physical and electrical connection to external apparatus, as was the case in FIGURE 6, but also as means for withdrawing the transducers from the probe.

Since the directional indications produced through the
system of the present invention are dependent upon the
directional orientation of detection-transducers 203 and
204, it is preferred to provide indicia at the remote end
of support 202 which will enable the user to maintain the
desired orientation of support 202, and therefore, the
proper orientation of detector-transducers 203 and 204,
with respect to scan plane 205. It is within the scope of
the present invention, however, to collect additional
directional information from the detector-transducers by
changing the relative positions of these transducers with
respect to the scan plane, as for example by rotating
support 202 about its axis. By determining the orientations at which a given one of the detector-transducers no
longer is first insonified, it may be estimated that the
scan plane is located in the direction of an axis which
bisects these points. Alternatively, it is within the
scope of the present invention to provide more than 2
detector-transducers to provide additional directional
information. Particularly when the probe is a catheter,
such detector-transducers may be located along the body of
the probe to provide additional information concerning the
position and orientation of portions of the probe within
the body.

In FIGURE 11, the preferred embodiment B-mode imaging
system is illustrated which comprises a B-scan transducer
208 which is coupled to the body 200, and which ultrasonically illuminates an imaging field (perpendicular to the
plane of the paper) along the scan axis 205. It will be
understood that numerous such ultrasound imaging systems
are commercially available, and the principles of the
present invention are not limited to any particular one,
or to any particular operational mode of transducer 208,
whether transducer motion, transducer signal phasing,
combination of these, or the like should be employed.

Likewise, it is understood that signals to and from the transducer 208 may be coupled with the body 200 in any of a variety of well-known ways, including water paths, gel-type coupling media, and the like. As shown in FIGURE 11, needle 201 has penetrated the body 200 and the point thereof, including transducers 203 and 204 are located in the vicinity of, but not within the scan plane 205. Nonetheless, transducers 203 and 204 will receive some ultrasonic waves from transducer 208 which will result in electrical signals being transferred along connecting cables 206 to sequence logic 207.

Sequence logic 207 distinguishes which of detector-transducers 203 and 204 is first insonified. This determination permits the user to ascertain the relative location of the scan plane 205 with respect to the detector-transducers 203 and 204, thereby permitting an adjustment in the scan plane location, and/or in the location of transducers 203 and 204. In order to readily facilitate such relative repositioning of these components, a direction indicator 209 is provided which responds to the output of the sequence logic to inform the operator of the sensed position of the detector transducers with respect to the scan plane. In the preferred embodiment, the direction indicators may be audible tones, lights, or other indicia which permit the user to reposition the components of the system in response thereto.

In FIGURE 13 a preferred sequence and detection indicator logic is illustrated wherein inputs from the left and right transducers are subjected to conventional signal conditioning circuits 301 and 303. Such signal conditioning routinely comprises amplification of the detector-transducers signals together with noise rejection conditioning, such as the noise rejection circuitry disclosed in connection with the point-source transducers of aforementioned U.S. Patent No. 4,249,539. The output of these signal conditioning circuits then passes to a phase comparator 305 which selectively activates move-left or move-right indicators 307 or 309 in response to the sensed first arrival of signals at the left or right transducer. A bistable flip-flop 313 ensures that the appropriate indicator remains activated until a changed arrival sequence is detected. The preferred phase comparator is a comparator capable of determining the "lead" or "lag" phase relationship, and the time difference between the leading edges of the wave forms. Using such a comparator, the U output is high when the wave form detected from the left transducer leads the wave detected from the right transducer. When the wave form detected at the right transducer leads the wave form detected from the left transducer, the D output is high, resulting in the activation of move right indicator 309. Such a comparator can detect a 3 nanosecond timing difference (delta t), which is equivalent, at an operating frequency of 3 MHz, to about 0.01 wave length. For transducers separated by 0.005 inches, it has been estimated that this degree of sensitivity will permit detection of an offset of about 2.4 mm when the needle is 6 cm deep in the body.

In view of the foregoing description, those of ordinary skill in the art will recognize that the particular signal conditioning circuits 301 and 303 selected for use in the sequence logic should preferably condition the wave form of the received signal to provide a reliable wave front to comparator 313. In such instances, for example, signal conditioning may comprise differentiating the signals received from the transducers to produce spiked wave forms for transmission to comparator 313. In FIGURE 13, representative square wave signals offset by a time (delta t) are illustrated for the phase comparator input, which, as illustrated, result in a "high" D output. The signal conditioned outputs from the left and right transducers is also provided through "or" gate 311 to the imaging control 211 illustrated in FIGURE 11. In this respect, once the detector-transducers are disposed, within the scan plane 205, these transducers may be utilized as if they were a single, omni-directional point-source transducer such as disclosed in U.S. Patent No. 4,249,539. As such, the location of the tip of needle 201 may be displayed at display 213, which also displays the image which has been derived from the imaging control 211 in combination with pulse transmitter 215, pulse receiver 217, and the aforementioned B-scan transducer 208.

In accordance with an alternate embodiment of the present invention the sequence logic 207 will time the delay between the receipt of wave arrivals at the detector-transducers. Since different time delays will be experienced with different probe-scan area orientations, such time delays may be correlated to the relative angle between the detector-transducers and the scan area. As the number and distance between detector-transducers increase, the desirability of timing delays between different detector-transducers to obtain further information concerning the direction of the scan plane increases.

As seen from the above, the present invention provides a novel approach to aiding a physician in guiding a tissue probe with respect to an ultrasound scan area by providing locational and directional information which readily enables the tissue probe to be positioned within a scan area.

Claims

1. In a system for deriving an image of a region of a body by applying ultrasound energy to the region by means of an external transducer probe locator apparatus comprising:

    a) hollow needle means for insertion into the body;

    b) conduit means, carried within said needle means, for detecting ultrasound energy near the tip region of said needle means and for conveying said energy through said needle means and outside the body; and

    c) means coupled to receive energy from said conduit means outside the body.

2. Apparatus as described in claim 1 wherein said conduit means comprises a solid, sonically conductive rod extending inside said needle means and terminating in an oblique surface cross-section.

3. Apparatus as described in claim 2 wherein said conduit means includes a matching layer, attached to said cross-section, for coupling incident sonic energy to said rod.

4. Apparatus as described in claim 2 wherein said conduit means forms an annular liquid tight seal near the inserted extremity of said needle means.

5. Apparatus as described in claim 2 wherein said conduit means comprises plural solid rods carried within said needle means and extending respective plural different distances into the body, thereby providing means for sensing the timed receipt of sonic pulses at said distances, and hence the direction and propagation speed of sonic energy in the body.

6. Apparatus as described in claim 2 wherein said rod makes periodic, relatively short contacts with the inner surface of said needle means.

7. Apparatus as described in claim 6 wherein said rod defines periodic annular ridges spanning a gap between said rod and said needle means.

8. Apparatus as described in claim 6 wherein said rod is helical in configuration, thereby making periodic contact with the inner surface of said needle means.

9. Apparatus as described in claim 1, further comprising acoustic insulation of said conduit means from the means for inner surface of said needle means, wherein said means for insulation comprises a packing of microspheres in the void between said conduit means and the inner surface of said needle means.

10. Insulation means as described in claim 9 wherein said microspheres are hollow, composed of plastic material, and sized in the range less than 0.1 mm. diameter.

11. Insulation means as described in claim 10 wherein said conduit means is composed of solid, sonically conductive material, and said microspheres are bonded to said conduit menas and to one another by low viscosity, sonically non-conductive epoxy.

12. Apparatus as described in claim 1, wherein:

said conduit means includes a plurality of detector transducers disposed at selected locations near the tip region of said needle means;

said energy receiving means comprises circuit means electrically coupled to said detector transducers for sensing the sequence of arrival of ultrasonic waves at said locations; and further comprising

a pulse-echo ultrasound imaging system for imaging a scan area of the body which includes said selected locations, said imaging means including an imaging transducer for acoustic coupling to the exterior of the body, whereby the relative directional position of said hollow needle means with respect to said scan area may be determined.

13. The apparatus of claim 17 further comprising indicator means for indication of said relative directional position.

14. The apparatus of claim 13 wherein said circuit means comprises a phase comparator means for determining the sequence of arrival of said waves.

15. The apparatus of claim 14 wherein said circuit means further comprises bistable means driven by said comparator means for selectively maintaining the relative directional indication of said indicator means until a different arrival sequence is detected by said phase comparator.

Fig.1.

Fig.2.

Fig.3.

Fig.4.

Fig.5.

Fig.6.

## Fig.7A.

## Fig.7B.

## Fig.8A.

## Fig.8B.

**Fig.9.**

501    511    502    509    507

505    510

**Fig.10.**

501    511    507    502    509

SONIC ENERGY

Fig.11

213
DISPLAY

209
DIRECTION
INDICATOR

217
PULSE
RECEIVE

211
IMAGING
CONTROL

215
PULSE
TRANSMIT

208
B-SCAN TRANSDUCER

207
SEQUENCE
LOGIC

BODY
200

202

201

205

203
204

206

Fig.12.

202

A

204    203

Fig.13.

FROM LEFT TRANSDUCER

SIGNAL CONDITIONING  301

303  SIGNAL CONDITIONING

FROM RIGHT TRANSDUCER

"OR" GATE

TO IMAGING SYSTEM

$\Delta t$

305

$\bar{U}$

$\bar{D}$

PHASE COMPARATOR

307  "MOVE LEFT" INDICATOR

309  "MOVE RIGHT" INDICATOR

313

# EUROPEAN SEARCH REPORT

European Patent Office

Application number

## DOCUMENTS CONSIDERED TO BE RELEVANT

EP 83300061.5

| Category | Citation of document with indication, where appropriate, of relevant passages | Relevant to claim | CLASSIFICATION OF THE APPLICATION (Int. Cl. ³) |
|---|---|---|---|
| X | GB - A - 1 462 147 (AKADEMIET FOR DE T.V.S.) <br> * Totality * | 1,2 | A 61 B 10/00 |
| A | | 3,12 | |
| X,D | US - A - 4 249 539 (D.H. VILKO-MERSON et al.) <br> * Fig. 1-3; column 2, line 46 - column 4, line 54 * | 1 | |
| A | | 12 | |
| A | GB - A - 1 298 707 (SIEMENS AG) <br> * Fig. 1; page 2, lines 66-91 * | 1,2 | |
| D,A | US - A - 3 556 079 (H. OMIZO) <br> * Fig. 4b,8c; column 3, lines 8-11; column 4, lines 6-23 * | 1,2 | **TECHNICAL FIELDS SEARCHED (Int. Cl. ³)** <br><br> A 61 B |
| A | GB - A - 2 073 418 (GOVERNING COUNCIL UN.) <br> * Fig. 1,2; page 1, line 102 - page 2, line 4 * | 1,12 | |

The present search report has been drawn up for all claims

| Place of search | Date of completion of the search | Examiner |
|---|---|---|
| VIENNA | 20-04-1983 | LUDWIG |